# EUROPEAN PATENT APPLICATION

(11) **EP 4 620 498 A1**
(43) Date of publication of application: **24.09.2025**
(21) Application number: 24305429.3
(22) Date of filing: 22.03.2024
(51) Int. Cl.: A61M 5/00, A61M 5/32, B65B 5/06, B65D 30/08, A61M 39/10, A61M 39/12

(54) **AN ASSEMBLY FOR STORING LOOSE ITEMS SUCH AS STOPPERS FOR SYRINGES**

(71) Applicant: Becton Dickinson France, 38800 Le Pont-de-Claix (FR)
(72) Inventor: POTEREAU, Emelyne, 38500 Voiron (FR); BELLE, Delphine, 38410 Saint-Martin-d'Uriage (FR); BALASOUPRAMANIANE, Gayatri, 38100 Grenoble (FR)
(74) Representative: Germain Maureau

(57) **Abstract**

The assembly (1) comprises a container (10) which comprises:
- a flexible body (20) including a first sheet (21) and a second sheet (22) secured to one another along part of their corresponding their edges, the body (20) forming an internal volume and having a filling aperture and an exit opening for the stored items to exit the container;
- a rigid connector (40) mounted at the exit opening.

The connector is placed relative to the body with its axis (A40) orthogonal to the sheets (21, 22), the connector being located on the side of the first sheet (21). A region of the body located around the connector is wrapped around the inner part of the connector, from the side of the second sheet (22). Furthermore, a tie (54) is removably placed around the connector from the side of the second sheet, the tie thereby also surrounding said region of the body and tightening it against and around the connector.

Thus, there is formed in the body and around the connector inner part (45) an enclosed area (33) into which the items cannot enter as long as the tie remains in position.

## Description

The invention relates to an assembly for storing loose items such as stoppers for syringes, and to a method for making such an assembly.

The invention applies in particular to containers used to transfer stoppers (also called plunger stoppers) or other items into a sterile enclosure called isolator. Such a container comprises a flexible body and a rigid connector mounted at an opening of the body. The stoppers are introduced in the container through a filling aperture which is subsequently closed. This technology is usually referred to as "rapid transfer port" (RTP).

The connector, which is usually referred to as beta port, is configured to cooperate with a door of the isolator, which is usually referred to as alpha port, and, for that purpose, is provided with specific functional parts that include ribs and form small compartments. As a consequence, the stoppers can be stuck in the connector, which is not desirable for several reasons:
- the stuck stoppers can prevent an appropriate operation of the whole system, for example by preventing the cooperation between the connector and the isolator door, especially when a rigid sleeve is involved in the process;
- some of the stuck stoppers are eventually freed but can be damaged or deformed, resulting in a loss of performances;
- some stoppers remain trapped in the connector, and therefore are lost and cannot be used.

An object of the invention is to improve such containers to prevent stored items from being stuck in the connector, or at least to limit their number.

For that purpose, the invention relates to an assembly for storing loose items such as stoppers for syringes, the assembly comprising a container which comprises:
- a flexible body including a first sheet and a second sheet, said sheets having substantially the same shape, being superimposed on each other and secured along at least part of their corresponding edges so that the body forms an internal volume, the body having a filling aperture which is open before and during the filing of the container with the items, and an exit opening for the stored items to exit the container;
- a rigid connector mounted at the exit opening and secured to the body, the connector having an axis, an inlet, an outlet and, with respect to the axis and to the path direction of the items exiting the container, an inner part and an outer part;
wherein the connector is initially secured to the body or subsequently placed relative to the body so as to be arranged with its axis substantially orthogonal to the sheets, the connector being located on the side of the first sheet, a region of the body located around the connector being wrapped around the inner part of the connector, from the side of the second sheet, and wherein the assembly further comprises a tie removably placed around the connector from the side of the second sheet, the tie thereby also surrounding said region of the body and tightening it against and around the connector, whereby there is formed in the body an enclosed area around the connector inner part, into which enclosed area the items cannot enter as long as the tie remains in position.

In other words, the tie locally presses the sheets the one towards the other, thus forming the boundary of an enclosed area in which the items cannot enter. As the wrapped region of the body is sandwiched between the connector and the tie, said enclosed area is arranged around the connector inner part. As a result, the items cannot reach the connector and therefore cannot be stuck in the connector. The enclosed area acts as a buffer zone, as long as the tie is not removed.

The tie can comprise an elastic band, which may be substantially a closed circular loop.

In an embodiment, the assembly further comprises a cap removably mounted around the connector. The tie may be attached to the cap. One advantage of this arrangement is to limit the risks of the tie being lost. Besides, this makes the removal of the cap and tie easier.

The cap may comprise a flexible wall having an edge, as well as an elastic band attached at said edge and configured to tightly surround the connector on the side of the connector outer part. The tie may be attached to the cap near said edge. Owing to this arrangement, the tie is easily accessible to be manipulated.

In an embodiment, the exit opening is formed between two corresponding edges of the first and second sheets, at one end of the body, the connector being secured to both sheets, with the connector axis being substantially parallel to said sheets in a neutral state of the container. Furthermore, in a ready-to-fill state, the body is folded so that said end of the body is located on the rest of the body, on the first sheet, and so that the connector is placed relative to the body with its axis substantially orthogonal to the sheets.

In a variant, the exit opening could be arranged in one sheet, preferably at a distance from the end of the body, the connector being mounted in said exit opening with its axis substantially orthogonal to the sheets, both in the neutral state and in the ready-to-fill state.

The body may contain loose items, such as stoppers for syringes, the enclosed area not containing such loose items. The filling aperture can be closed.

The invention also relates to a method for making an assembly for storing loose items such as stoppers for syringes. The method comprises:
a) providing a container which comprises:
   - a flexible body including a first sheet and a second sheet, said sheets having substantially the same shape, being superimposed on each other and secured along at least part of their corresponding edges so that the body forms an internal volume, the body having a filling aperture which is open before and during the filing of the container with the items, and an exit opening for the stored items to exit the container;
   - a rigid connector mounted at the exit opening and secured to the body, the connector having an axis, an inlet, an outlet and, with respect to the axis and to the path direction of the items exiting the container, an inner part and an outer part;
b) placing the connector relative to the body so that the connector axis is substantially orthogonal to the sheets and the connector is located on the side of the first sheet, if the connector is not initially arranged in said position;
c) wrapping a region of the body located around the connector around the inner part of the connector, from the side of the second sheet;
d) removably placing a tie around the connector from the side of the second sheet, the tie thereby also surrounding said region of the body and tightening it against and around the connector, whereby there is formed in the body an enclosed area around the connector inner part, into which enclosed area the items cannot enter as long as the tie remains in position.

The method may further comprise removably mounting a cap around the connector, preferably before step c), or before step b).

In an embodiment in which the exit opening of the body is formed between two corresponding edges of the first and second sheets, at one end of the body, the connector being secured to both sheets, with the connector axis being substantially parallel to said sheets in a neutral state of the container, step b) of the method may comprise folding the body so that said end of the body is located on the rest of the body, on the first sheet, and so that the connector is placed relative to the body with its axis substantially orthogonal to the sheets in a ready-to-fill state.

The method may further comprise, before step b) or after step d):
e) introducing loose items in the body by the filling aperture;
f) closing the filling aperture, preferably by securing the first sheet and the second sheet to one another along corresponding edges.

A possible embodiment of the invention will now be described by way of a non-limiting example with reference to the appended figures:
Figure 1 is a plan view of a container comprising a flexible body and a rigid connector and being in a neutral state;

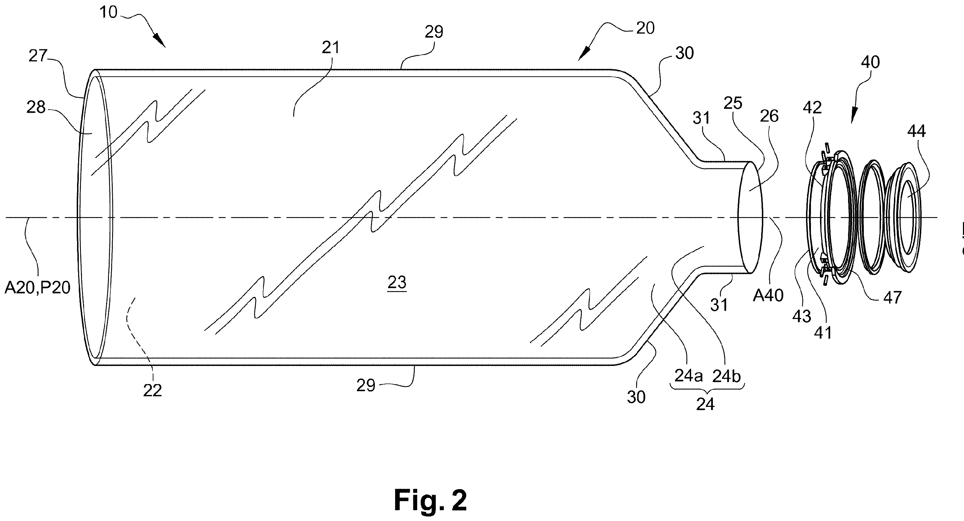

Figure 2 is a view similar to figure 1, which the connector not secured to the body and illustrated in an exploded perspective view;
Figure 3 is a plan view of a cap to be mounted on the connector, the cap having a tie attached thereto;
Figure 4a shows a step of a method according to an embodiment of the invention, for making an assembly in a ready-to-fill state or ready-to-use state from the container in the neutral state shown in figure 1 and the cap shown in figure 3;
Figure 4b is a view of the assembly after the step shown in figure 4a;
Figure 5a shows a subsequent step of the method;
Figure 5b is a view of the assembly after the step shown in figure 5a;
Figures 6a and 6b show a subsequent step of the method;
Figure 6c is a view of the assembly after the step shown in figures 6a and 6b, the assembly being in the ready-to-fill state;
Figure 7 is a view similar to figure 6c, seen from another side;
Figure 8 shows a subsequent step in which loose items are introduced in the assembly of figure 7;
Figure 9 is a view of the assembly after the step shown in figure 8, the assembly being in the ready-to-use state.

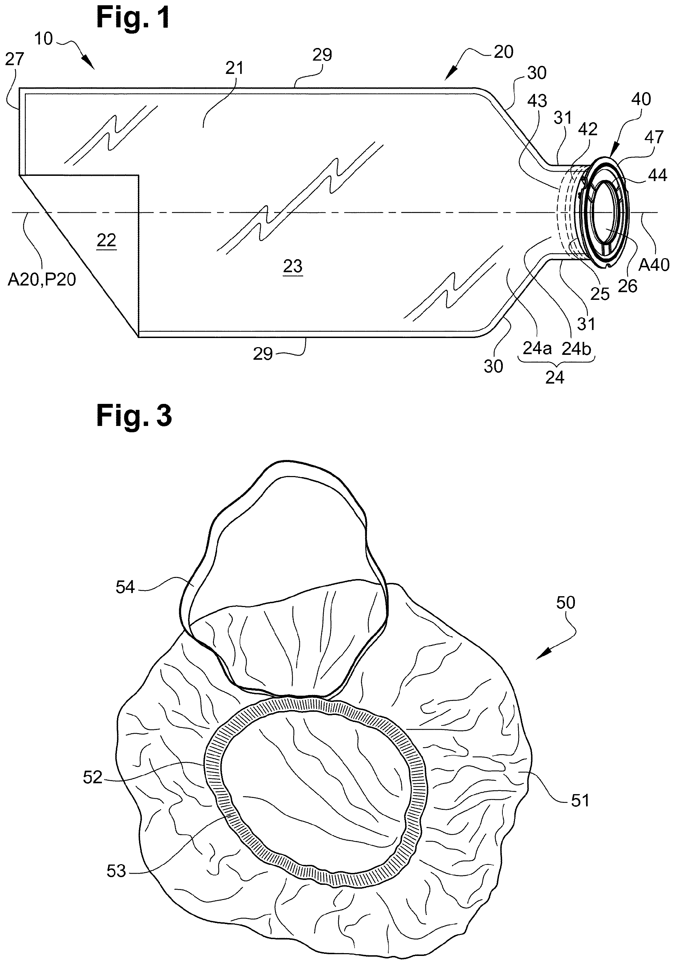

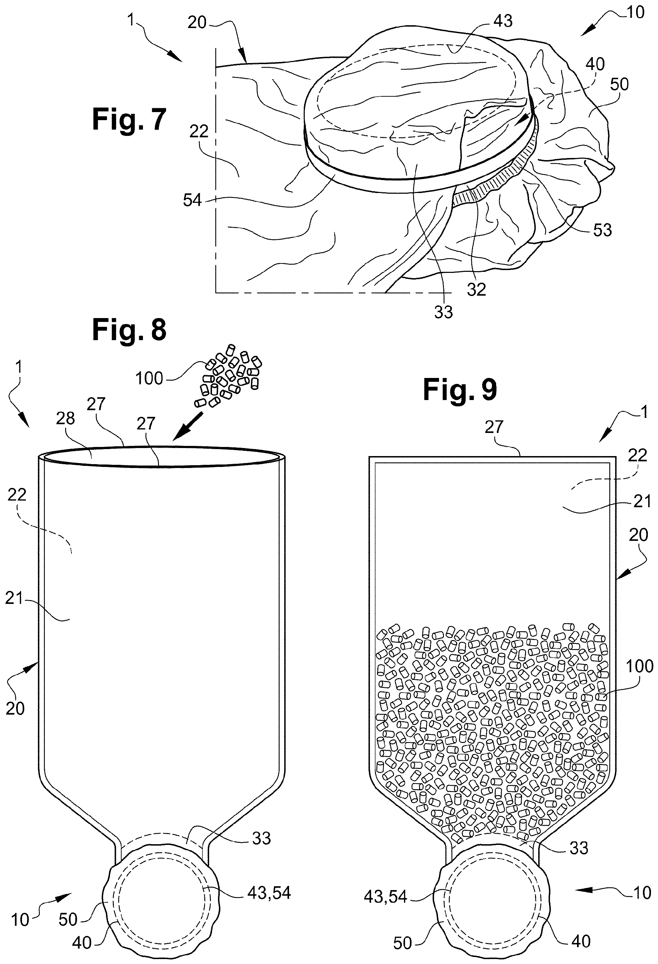

Figure 1 shows a container 10 which is configured to be partly filled with loose items such as stoppers 100 for syringes, as illustrated in figures 8 and 9.

The container 10 comprises a flexible body 20 which includes a first sheet 21 and a second sheet 22 and forms an internal volume. The sheets 21, 22 substantially have the same shape and are superimposed on each other.

In figure 1, the container 10 is in a neutral state, i.e. in a position in which the flexible body 20 rests on a flat surface without being subjected to any mechanical stress, the sheets 21, 22 being parallel to said flat surface. It has to be noted that one corner is turned-up only for description purposes. In an embodiment, the body 20 defines a longitudinal axis A20 that is substantially parallel to the sheets 21, 22 in the neutral state, and a median plane of symmetry P20 which is orthogonal to the sheets 21, 22 and includes said longitudinal axis A20.

In the non-limiting illustrated embodiment, the first sheet 21 and the second sheet 22 have a substantially rectangular main portion 23 and an end portion 24, said end portion 24 having a converging section 24a extending from the main portion 23 and a neck section 24b extending from the converging section 24a, opposite the main portion 23.

The sheets 21, 22 are secured along all their corresponding edges except:
- one first set of edges called foremost edges 25, thereby forming between them an exit opening 26 of the body 20 for the stored stoppers 100 to exit the container 10; These foremost edges 25 are preferably located at the free end of the neck section 24b;
- and second one set of edges, called bottom edges 27, preferably opposite the foremost edges 25, thereby forming a filling aperture 28. These bottom edges 27 are preferably located at the free end of the main portion 23. The filling aperture 28 is open before and during the filing of the container 10 with the stoppers 100, and subsequently closed when the container 10 is in a ready-to-use state.

In addition to the bottom edge 27 and the foremost edge 25, each sheet 21, 22 has two lateral edges 29 substantially parallel to the longitudinal axis A20, on each side of the main portion 23, two converging edges 30 on each side of the converging section 24a, and two end side edges 31 on each side of the neck section 24b.

In an embodiment, the first sheet 21 is made of a plastic material such as HDPE (high-density polyethylene), and the second sheet 22 made of Tyvek^{®}, or of another porous material enabling sterilization and preventing contamination. The opposite configuration is also possible. In other embodiments, the first sheet 21 and second sheet 22 can be made in PE (polyethylene), LDPE (low density polyethylene), HDPE (high-density polyethylene) PA (polyamide), in a three-layered material PE/PA/PE (Polyethylene, Polyamide, polyethylene) or in a three-layered material PE (LDPE/HDPE/LDPE).

The corresponding edges 27, 29, 30, 31 of the sheets 21, 22 may be secured to one another by welding.

The container 10 also comprises a rigid connector 40 which is mounted at the exit opening 26 and secured to the body 20. For example, the connector 40 comprises an external sheath 41 provided with an external ring 42, and the neck sections 24b of the sheets 21, 22 are placed outside the external sheath 41 and secured to the external ring 42, i.e. around the connector 40. This should however not be considered as limiting.

The connector has an axis A40 which is substantially parallel to the sheets 21, 22 in the neutral state of the container 10, and substantially coincident with the longitudinal axis A20 of the body 20.

The term "internal" is used for an element located closer to axis A20 or A40 than an "external" element. The terms "inner" and "outer" are used with respect to the axis and to the path direction of the stoppers 100 exiting the container 10.

The connector 40 has an inlet 43 and an outlet 44. The outlet 44 is closed by a wall until the container 10 is attached to an isolator for the transfer of the stoppers 100 from the container 10 to the isolator.

The connector 40 further has:
- an inner part 45, which is a part of the connector 40 located on the side of the inlet 43 i.e. on the side of the body 20; in the disclosed embodiment, the inner part 45 is at least partially located between the sheets 21, 22, i.e. inside the body 20, but other embodiments are possible;
- and an outer part 46, which is a part of the connector 40 located on the side of the outlet 44.

The connector 40 may further comprise an external collar 47 projecting from the external sheath 41 substantially at the outlet 44. The connector 40 may also comprise an internal sleeve (not shown) movable axially relative to external sheath 41, the function of which is to prevent contamination of the transfer of the stoppers 100 from the container 10 to the isolator.

Figure 3 shows a cap 50 intended to be removably mounted around the connector 40, in order to protect the connector 40 from contaminants such as dust.

In the illustrated embodiment, the cap 50 comprises a flexible wall 51 that is for example made of a Tyvek^{®} sheet, or of another porous material enabling sterilization and preventing contamination. The flexible wall 51 initially has substantially the form of a disc with an edge 52 that is peripheral and circular. An elastic band 53 is mounted at said edge 52 and forms a closed loop. In a rest state, i.e. when it is not elongated, the elastic band 53 has a diameter that is lower than the diameter of the disc forming the initial shape of the flexible wall 51. As a result, the flexible wall 51 does not remain planar but becomes shaped as a shower cap.

Furthermore, a tie 54 is attached to the cap 50, preferably at or near said edge 52. The tie 54 can be an elastic band forming a closed loop.

The tie 54 and elastic band 53 preferably have substantially the same diameters in their rest states. Both are configured to be placed around the connector 40, to tightly surround it, as will be explained later. Here, the term "tightly" means that the elastic band / tie exert a radial clamping force which is sufficient to ensure that the cap / tie remains on the connector; however, no sealing is achieved, and the cap / tie remain easy to remove manually without requiring any tool.

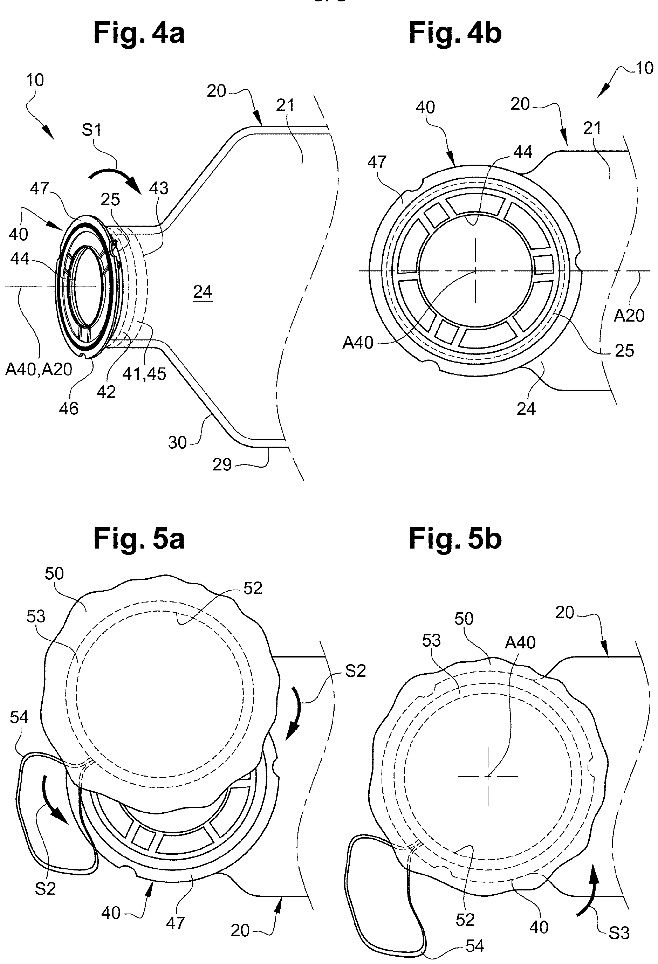

Reference is now made to figures 4a - 7 to describe a method for making an assembly 1 comprising the container 10 and the cap 50, or at least the tie 54, in which assembly the stoppers 100 are prevented from being stuck in the connector 40. Although the steps of the method are described as being made by an operator, at least some of them can be made by a machine.

Starting from the neutral state of the container 10 shown on figure 1, and as illustrated by arrow S1 in figure 4a, the connector 40 is placed relative to the body 20 so that the connector axis A40 is substantially orthogonal to the sheets 21, 22 and the connector 40 is located on the side of the first sheet 21.

In practice, this is achieved by folding the body 20 upon itself.

More specifically, the body 20 is folded so that the foremost edges 25 become parallel to the first sheet 21, and at least partially located on the end portion 24. In other words, the connector 40 is substantially rotated about an axis which is included in the plane of the first sheet 21 and orthogonal to the longitudinal axis A20, so that the connector inlet 43 rests on the first sheet 21 and so that the connector axis A40 is substantially orthogonal to the sheets 21, 22.

The container 10 in the configuration after this step of the method is illustrated in figure 4b.

Then, the cap 50 is put in place. For that purpose, and as shown by arrows S2 in figure 5a, an operator takes the cap 50, expands the elastic band 53, places the cap 50 over the connector 40 from the side of the outlet 44 and past the external collar 47, with the elastic band 53 around the external sheath 41. Then, the operator releases the elastic band 53 which thus returns towards its rest state and tightly surrounds the connector 40, on the side of the connector outer part 46.

The container 10 in the configuration after this step of the method is illustrated in figure 5b. as can be seen, the tie 54 remains free - except for its part attached to the cap 50. The tie 54 preferably extends opposite the body 20.

It has to be noted that, alternatively, the cap 50 can be mounted on the connector 40 prior to placing the connector 40 such that its axis A40 is substantially orthogonal to the sheets 21, 22. Whatever the respective order of these first two steps of the method, the resulting configuration of the container 10 is the same.

Then, the container 10 equipped with the cap 50 as shown in figure 5b is turned, according to arrow S3, so that the operator can access the other side of the container 10, i.e. the side of the second sheet 22.

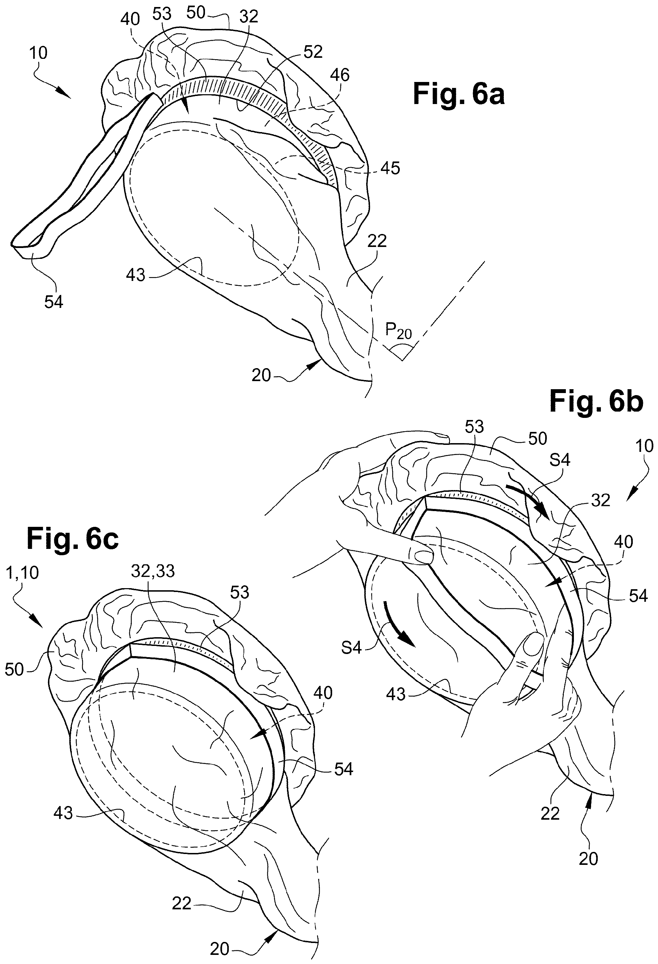

A region 32 of the body 20 which is located around the connector 40 is then wrapped by the operator around the inner part 45 of the connector 40, from the side of the second sheet 22, leading to the configuration shown in figure 6a.

The operator then puts the tie 54 in place as represented by arrows S4 in figure 6b. The operator enlarges the tie 54 radially and places it around the connector 40, from the side of the second sheet 22, the tie 54 thereby also surrounding the region 32 of the body 20 which is wrapped around the inner part 45 of the connector 40. Once the operator releases the tie 54, the tie 54 returns towards its rest state and tightens said region 32 of the body 20 against and around the connector 40.

As a result, there is formed in the body 20 an enclosed area 33 around the connector inner part 45, which is separated from the rest of the body 20 by the tie 54, which acts as a boundary. Such enclosed area 33 is also schematically illustrated in figures 8 and 9.

The assembly 1 obtained after this step is illustrated in figures 6c and 7, viewed according to different orientations.

The stoppers 100 - or other loose items - are then introduced in the body 20, by the filling aperture 28. Owing to the tie 54 and its arrangement around the connector 40 and region 32, the stoppers 100 cannot enter the enclosed area 33. The risk of stoppers 100 being stuck in the connector 40 is therefore avoided.

When the body 20 is filled with the desired amount of stoppers 100, the filling aperture 28 is closed. To that end, the bottom edges 27 of the first sheet 21 and the second sheet 22 are secured to one another. The assembly 1 thus obtained is illustrated in figure 9. As can be seen, no stopper 100 is located in the enclosed area 33. The container 10 and assembly 1 are in a ready-to-use state and can be delivered to the customer facility in which the isolator is located, for the stoppers 100 to be used in a subsequent sterile assembly line.

The invention allows improving the current assemblies and methods for making these assemblies by the implementation of an additional component, namely the tie 54, that is neither heavy nor expensive.

The invention is further advantageous in that it does not involve any additional step for the customer, except the removal of the tie 54. This step is very easy and fast, and can be completed together with the removal of the cap 50.

The invention is not limited to the embodiment described above by way of example but it rather comprises all the technical equivalents and variants of the means described as well as their combinations.

It is to be understood that the present invention is not limited to the embodiments described above and illustrated in the drawings; rather, the skilled person will recognize that many changes and modifications may be made within the scope of the appended claims.

## Claims

1. An assembly (1) for storing loose items such as stoppers (100) for syringes, the assembly (1) comprising a container (10) which comprises:
- a flexible body (20) including a first sheet (21) and a second sheet (22), said sheets (21, 22) having substantially the same shape, being superimposed on each other and secured along at least part of their corresponding edges so that the body (20) forms an internal volume, the body (20) having a filling aperture (28) which is open before and during the filing of the container (10) with the items, and an exit opening (26) for the stored items to exit the container (10);
- a rigid connector (40) mounted at the exit opening (26) and secured to the body (20), the connector (40) having an axis (A40), an inlet (43), an outlet (44) and, with respect to the axis (A40) and to the path direction of the items exiting the container (10), an inner part (45) and an outer part (46);
wherein the connector (40) is initially secured to the body (20) or subsequently placed relative to the body (20) so as to be arranged with its axis (A40) substantially orthogonal to the sheets (21, 22), the connector (40) being located on the side of the first sheet (21), a region (32) of the body (20) located around the connector (40) being wrapped around the inner part (45) of the connector (40), from the side of the second sheet (22),
and wherein the assembly (1) further comprises a tie (54) removably placed around the connector (40) from the side of the second sheet (22), the tie (54) thereby also surrounding said region (32) of the body (20) and tightening it against and around the connector (40), whereby there is formed in the body (20) an enclosed area (33) around the connector inner part (45), into which enclosed area (33) the items cannot enter as long as the tie (54) remains in position.

2. The assembly according to claim 1, wherein the tie (54) comprises an elastic band.

3. The assembly according to claim 1 or claim 2, further comprising a cap (50) removably mounted around the connector (40), and wherein the tie (54) is attached to the cap (50).

4. The assembly according to any claim 3, wherein the cap (50) comprises a flexible wall (51) having an edge (52), the cap (50) further comprising an elastic band attached at said edge (52) and configured to tightly surround the connector (40) on the side of the connector outer part (46) and wherein, preferably, the tie (54) is attached to the cap (50) near said edge (52).

5. The assembly according to any one of claims 1 to 4, wherein the exit opening (26) is formed between two corresponding edges (25) of the first and second sheets (21, 22), at one end of the body (20), the connector (40) being secured to both sheets (21, 22), with the connector axis (A40) being substantially parallel to said sheets (21, 22) in a neutral state of the container (10), and wherein, in a ready-to-fill state, the body (20) is folded so that said end of the body (20) is located on the rest of the body (20), on the first sheet (21), and so that the connector (40) is placed relative to the body (20) with its axis (A40) substantially orthogonal to the sheets (21, 22).

6. The assembly according to any one of claims 1 to 5, wherein the body (20) contains loose items, such as stoppers (100) for syringes, the enclosed area (33) not containing such loose items, and wherein the filling aperture (28) is closed.

7. A method for making an assembly (1) for storing loose items such as stoppers (100) for syringes, the method comprising:
a) providing a container (10) which comprises:
- a flexible body (20) including a first sheet (21) and a second sheet (22), said sheets (21, 22) having substantially the same shape, being superimposed on each other and secured along at least part of their corresponding edges so that the body (20) forms an internal volume, the body (20) having a filling aperture (28) which is open before and during the filing of the container (10) with the items, and an exit opening (26) for the stored items to exit the container (10);
- a rigid connector (40) mounted at the exit opening (26) and secured to the body (20), the connector (40) having an axis (A40), an inlet (43), an outlet (44) and, with respect to the axis (A40) and to the path direction of the items exiting the container (10), an inner part (45) and an outer part (46);
b) placing the connector (40) relative to the body (20) so that the connector axis (A40) is substantially orthogonal to the sheets (21, 22) and the connector (40) is located on the side of the first sheet (21), if the connector (40) is not initially arranged in said position;
c) wrapping a region (32) of the body (20) located around the connector (40) around the inner part (45) of the connector (40), from the side of the second sheet (22);
d) removably placing a tie (54) around the connector (40) from the side of the second sheet (22), the tie (54) thereby also surrounding said region (32) of the body (20) and tightening it against and around the connector (40), whereby there is formed in the body (20) an enclosed area (33) around the connector inner part (45), into which enclosed area (33) the items cannot enter as long as the tie (54) remains in position.

8. The method according to claim 7, further comprising removably mounting a cap (50) around the connector (40), preferably before step c) or before step b).

9. The method according to claim 7 or claim 8, wherein the exit opening (26) of the body (20) is formed between two corresponding edges (25) of the first and second sheets (21, 22), at one end of the body (20), the connector (40) being secured to both sheets (21, 22), with the connector axis (A40) being substantially parallel to said sheets (21, 22) in a neutral state of the container (10), and wherein step b) of the method comprises folding the body (20) so that said end of the body (20) be located on the rest of the body (20), on the first sheet (21), and so that the connector (40) is placed relative to the body (20) with its axis (A40) substantially orthogonal to the sheets (21, 22)in a ready-to-fill state.

10. The method according to any one of claims 7 to 9, further comprising, before step b) or after step d):
e) introducing loose items in the body (20) by the filling aperture (28);
f) closing the filling aperture (28), preferably by securing the first sheet (21) and the second sheet (22) to one another along corresponding edges (27).
